# EUROPEAN PATENT SPECIFICATION

(11) **EP 3 332 695 B1**
(45) Date of publication and mention of the grant of the patent: **26.05.2021**
(21) Application number: 16203153.8
(22) Date of filing: 09.12.2016
(51) Int. Cl.: A61B 5/00

(54) **MEASUREMENT DEVICE**
MESSGERÄT
DISPOSITIF DE MESURE

(43) Date of publication of application: 13.06.2018
(73) Proprietor: Max-Planck-Gesellschaft zur Förderung der Wissenschaften e.V., 80539 München (DE)
(72) Inventor: Serwane, Friedhelm, 70569 Stuttgart (DE); Spatz, Joachim, 70569 Stuttgart (DE)
(74) Representative: Müller Hoffmann & Partner

(56) References cited:
- EP-A1- 3 190 176
- WO-A1-2015/121687
- WO-A1-2016/182969
- US-A1- 2005 059 148
- US-A1- 2016 116 394
- TERRENCE F. HOLEKAMP ET AL: "Fast Three-Dimensional Fluorescence Imaging of Activity in Neural Populations by Objective-Coupled Planar Illumination Microscopy", NEURON, vol. 57, no. 5, 1 March 2008 (2008-03-01), pages 661-672, XP055373407, US ISSN: 0896-6273, DOI: 10.1016/j.neuron.2008.01.011
- MISHA B AHRENS ET AL: "Whole-brain functional imaging at cellular resolution using light-sheet microscopy", NATURE METHODS, vol. 10, no. 5, 18 March 2013 (2013-03-18) , pages 413-420, XP055372957, ISSN: 1548-7091, DOI: 10.1038/nmeth.2434
- MANUELA VÖLKNER ET AL: "Retinal Organoids from Pluripotent Stem Cells Efficiently Recapitulate Retinogenesis", STEM CELL REPORTS, vol. 6, no. 4, 1 April 2016 (2016-04-01), pages 525-538, XP055372696, United States ISSN: 2213-6711, DOI: 10.1016/j.stemcr.2016.03.001
- Winfried Denk ET AL: "Optical recording of light-evoked calcium signals in the functionally intact retina Edited by", Neurobiology and approved April, 1 June 1999 (1999-06-01), pages 7035-7040, XP055373143, Retrieved from the Internet: URL:http://www.pnas.org/content/96/12/7035 .full.pdf [retrieved on 2017-05-16]
- UTO KOICHIRO ET AL: "Dynamically tunable cell culture platforms for tissue engineering and mechanobiology", PROGRESS IN POLYMER SCIENCE, PERGAMON PRESS, OXFORD, GB, vol. 65, 17 September 2016 (2016-09-17), pages 53-82, XP029897008, ISSN: 0079-6700, DOI: 10.1016/J.PROGPOLYMSCI.2016.09.004
- MADELINE A. LANCASTER ET AL: "Cerebral organoids model human brain development and microcephaly", NATURE, vol. 501, no. 7467, 28 August 2013 (2013-08-28), pages 373-379, XP055166627, ISSN: 0028-0836, DOI: 10.1038/nature12517
- XIUFENG ZHONG ET AL: "Generation of three-dimensional retinal tissue with functional photoreceptors from human iPSCs", NATURE COMMUNICATIONS, vol. 5, 4047, 10 June 2014 (2014-06-10), pages 1-14, XP055383383, DOI: 10.1038/ncomms5047 -& Xiufeng Zhong ET AL: "Supplementary figures and table. Generation of three-dimensional retinal tissue with functional photoreceptors from human iPSCs", NATURE COMMUNICATIONS, vol. 5, Article No.: 4047, online supplementary information, 10 June 2014 (2014-06-10), pages 1-6, XP055395634, Retrieved from the Internet: URL:https://images.nature.com/original/nat ure-assets/ncomms/2014/140610/ncomms5047/e xtref/ncomms5047-s1.pdf [retrieved on 2017-08-02]
- HOLLY YU CHEN ET AL: "Three-dimensional retinal organoids from mouse pluripotent stem cells mimic in vivo development with enhanced stratification and rod photoreceptor differentiation", MOLECULAR VISION, vol. 22, 9 September 2016 (2016-09-09), pages 1077-1094, XP055395173,

## Description

The present invention is concerned with measurement devices, that comprise neuronal tissue, in particular retinal tissue, grown from stem cells, in particular from induced pluripotent stem cells.

The retina plays a key role in enabling vision: After converting incoming photons into neuronal signals it processes and filters those in a highly parallel way using neuronal networks. For many species, including humans, those are formed by the five different types of retinal neurons including photoreceptors. Although experiments with animal retina explants have provided insights into principles of retinal signal processing as a function of the network parameters (neuron type, neuron density, neuron size and synaptic coupling strengths), those differ quantitatively across species. In particular, they have not been systematically quantified within human tissues, mainly due to the lack of availability of tissue explants and limitations in legally allowed genetic approaches.

Recently, organoids derived from human embryonic or induced pluripotent stem cells have found widespread attention due to their similarity in cell types and tissue morphology compared to their *in vivo* counterparts. However, it has not yet been possible to test the functionality of their neuronal networks. Similarly, it has not yet been possible to test the functionality of neuronal networks having a more complicated structure than retinal neuronal networks, such as the brain.

In order to (I) measure the function of the neuronal networks in retinal human tissue or other neuronal tissues and (II) generate a tunable neuronal network for signal processing, at least the following four outstanding problems need to be solved.

### Problem I: Human retina tissue composition, availability and accessibility to neuronal readout techniques

Measurements of the neuronal response within the entire human retina or even the human brain or spinal cord have not been performed due to severe experimental hurdles. A quantitative characterization of the neuronal networks requires the measurement of how a large number of individual neurons interact with their neighbors to generate a functional neuronal network. This requires the measurement of the neuronal responses (ms times resolution) within the entire network at single neuron resolution (10 microns spatial resolution). This combination of requirements exceeds state-of-the art capabilities. Hence, two model systems are used to study retinal neuronal networks.

On the one hand, retina tissue explants (e.g. mouse retina) are flattened to a 2D layer and mounted on top of electrode arrays to measure the neuronal signals (Gollisch, T., & Meister, M. (2010), "Eye Smarter than Scientists Believed: Neural Computations in Circuits of the Retina", Neuron, 65(2), 150-164). State-of-the-art devices allow the simultaneous readout of hundreds of neurons.

On the other hand, the neuronal responses of the entire brain have been recorded using animal models with small brains, mainly drosophila, zebrafish and C. elegans (Ahrens, M. B., & Engert, F., (2015), "Large-scale imaging in small brains", Current Opinion in Neurobiology, 32, 78-86; Borst, A., (2014), "Fly visual course control: behaviour, algorithms and circuits", Nature Publishing Group, 15(9), 590-599), because they can be imaged *in toto.*

Experiments with animal retinas and retina explants helped to obtain an understanding of retina function. However, the actual parameters of the neuronal networks show differences from animals to humans as they are optimized according to the specie's ecological niche and behavioral responses. For example, the synaptic coupling strength of the network is determined by the local dopamine concentration which is controlled by the distribution of dopaminergic amacrine cells (Bloomfield, S. A., & Völgyi, B., (2009), "The diverse functional roles and regulation of neuronal gap junctions in the retina", Nature Publishing Group, 10(7), 495-506). This distribution differs across the species (Törk, I. et al., (1995), "Neurotransmitters in the human brain", Plenum Press) which requires great care when transferring the knowledge of the neuronal networks obtained with animal models to the human.

Understanding the signal processing performed by the human retina or the human brain therefore requires experiments with a tissue which has similar neuronal network parameters. However, such tissue is not easily accessible in living humans and retinal explants are not easily available. Moreover, genetic modifications as a tool to probe and manipulate those networks are not available due to legal limitations.

Hence, there is a need for measurement devices that use specific forms of neuronal tissue, in particular of human retinal tissue, and methods for forming such retinal tissues in vitro.

### Problem II: Neuronal network tunability

Retinas or other neuronal tissues from humans and animals grow according to their genetic program which determines the parameters of their final neuronal network. Those are optimized according to the ecological niche of the species. The reproducibility of the neuronal network within a species comes to the cost of its tenability. This makes it technically very challenging to change network parameters (neuron type, size, density and coupling strengths). Although the synaptic coupling strength is controllable using opto-genetic techniques (Boyden, E. S. et al., (2005), "Millisecond-timescale, genetically targeted optical control of neural activity", Nature Neuroscience, 8(9), 1263-1268), the neuron size and density are particularly challenging to control. This limitation prevents the use of retinas and retina explants to design and explore custom neuronal networks for advanced signal processing applications. Researchers estimate that there are more than 30 different image processing filters implemented within the mouse retina given by the different neuron types and sizes. The ability to change these parameters will allow the implementation of custom processing algorithms with those networks.

Therefore, there is a need for measurement devices that allow understanding of the formation of neuronal networks within neuronal tissues such as the retina or the brain in a controllable manner. Moreover, there is a need for devices and methods that allow the tuning of growing neuronal networks such that they develop specific desired characteristics.

### Problem III: Organoid shape

In recent years, researchers were able to successfully grow organoids, i.e. organ-like structures with the same cell types and similar tissue morphologies as their *in vivo* counterparts, in Petri dishes. However, the overall shape of these organoids substantially varies from sample to sample. Even in state-of-the-art protocols the shape of the organoids is coarsely tuned by cutting them e.g. with forceps (Hiler, D. J. et al., (2016), "Reprogramming of mouse retinal neurons and standardized quantification of their differentiation in 3D retinal cultures", Nature Protocols, 11(10), 1955-1976). Their small size of about one millimeter makes this technique not feasible for creating arbitrary shapes.

As the function of an organ largely depends on the organ shape, in particular in the case of the brain and the retina, this prevents the controlled preparation of functional organs in vitro.

Thus, a need exists for devices and methods that allow the growing of organoid tissue in a predetermined form. The ability of the current invention to shape organoids in arbitrary morphologies has been developed in the context of the retina organoid, but can be used for other organoids as well, such as the brain organoid.

### Problem IV: 3D-readout of the neuronal responses

The retina, the retina organoids, and other neuronal tissues are in principle three-dimensional structures. Measuring the neuronal responses of multiple neurons in 3D is technically challenging. Therefore, experiments have been performed either using retina explants limited to 2D geometries (Obien, M. E. J., (2014), "Revealing neuronal function through microelectrode array recordings", 1-30) or small-brain animals such as drosophila, zebrafish and C. elegans in 3D (Ahrens, M. B., & Engert, F., (2015), "Large-scale imaging in small brains", Current Opinion in Neurobiology, 32, 78-86). It was not yet possible to perform a full 3D readout of the neuronal signals of a mm-sized retina or other neuronal tissue at cellular resolution.

"Fast Three-Dimensional Fluorescence Imaging of Activity in Neural Population by Objective-Coupled Planar Illumination Microscopy" by Holekamp T.F, et al. (NEURON, vol. 57, no. 5, 1 March 2008, pages 661-672) discusses the use of light sheet microscopy for imaging of large neuronal populations.

"Whole-brain functional imaging at cellular resolution using light-sheet microscopy" by Ahrens M.B., et al. (NATURE METHODS, vol. 10, no. 5, 18 March 2013, pages 413-420) discusses the use of light-sheet microscopy for imaging the entire brain of the larval zebrafish in vivo.

US 2005/059148 A1 discloses a cell culture system related to extended in vitro culture of mature retinal cells and methods for preparing the cell culture system. Also disclosed is a retinal cell culture stress model related to extended in vitro culture of mature retinal cells in the presence of a stressor and methods for using the cell culture stress model. A disclosed cell culture system comprises a long-term culture of mature retinal cells, without requiring addition of other types of non-retinal cells such as purified glia, or cells isolated from ciliary bodies within the eye, and the addition of a stressor such as light, A2E, cigarette smoke condensate, glutamate, or hydrostatic pressure. Methods for identifying bioactive agents that alter viability, neurodegeneration, or survival of retinal cells using the retinal cell culture stress system are also provided.

"Retinal Organoids from Pluripotent Stem Cells Efficiently Recapitulate Retinogenesis" by Völkner M, et al. (Stem Cell Reports, vol. 6, no. 4, 1 April 2016, pages 525-538) discloses a protocol for the efficient generation of large, 3D-stratified retinal organoids.

"Optical recording of light-evoked calcium signals in the functionally intact retina" by Denk W. and Detweiler P.B. (Proc. Natl. Acad. Sci. USA, Neurobiology, vol. 96, June 1999, pages 7035-7040) discloses the use of two-photon excitation of fluorescent indicator dyes to measure calcium concentration transients in retinal cells.

"Dynamically tunable cell culture platforms for tissue engineering and mechanobiology" by Uto K., et al. (Progress in Polymer Science, Pergamon Press, Oxford, GB, vol. 65, 17 September 2016, pages 53 to 82) reviews in vitro efforts to mimic the dynamic microenvironment comprising native tissue extracellular matrix from the viewpoint of material design and efforts in tissue engineering.

"Cerebral organoids model human brain development and microcephaly" by Lancaster M.A., et al. (NATURE, vol. 501, no. 7467, 28 August 2013, pages 373-379) discusses the development of human pluripotent stem cell-derived three-dimensional organoid culture systems that develop various discrete, although interdependent brain regions.

"Generation of three-dimensional retinal tissue with functional photoreceptors from human iPSCs" by Zhong X., et al. (nature COMMUNICATIONS, vol. 5, 10 June 2014) discusses formation of three-dimensional retinal cups that contain all major retinal cell types arranged in their proper layers from induced pluripotent stem cells.

WO 2016/182969 A1 discloses a three-dimensional cell growth medium. The cell growth medium may comprise hydrogel particles swollen with a liquid cell growth medium to form a granular gel yield stress material which undergoes a phase transformation from a solid phase to a liquid- like phase when an applied stress exceeds the yield stress. Cells may be placed in the three- dimensional cell growth medium according to any shape or geometry, and may remain in place within the three-dimensional cell growth medium.

In view of the prior art it is a need to provide measurement devices that allow a readout as described above.

The present invention provides solutions to these problems based on the following guiding principles.

(a) Human-like neuronal or retinal tissues may be grown from human embryonic or induced pluripotent stem cells. It is possible to successfully grow retina organoids with functional photoreceptor cells from human induced pluripotent stem cells. Here, the neuron types within the system can be controlled in a simple way by the application of specific transcription factors during retina development. This is for example described in Zhong, X. et al.: "Generation of three dimensional retinal tissue with functional photoreceptors from human iPSCs" (Nature Communications, 5, 4047 EP-, 2014), Völkner, M. et al.: "Retinal Organoids from Pluripotent Stem Cells Efficiently Recapitulate Retinogenesis" (Stem Cell Reports, 6(4), 525-538, 2016), Eiraku, M. et al.: "Self-organizing optic-cup morphogenesis in three-dimensional culture" (Nature 472, 51 - 56, 2011), WO 2015/109148 A1, CN 103 409 363 A, and US 2016/0333312 A1.

(b) The shape of the organoid may be controlled by embedding the developing organoid, such as the retina organoid or another neuronal organoid, in an environment with controlled mechanical properties. By a local softening or stiffening of the environment as previously described (Stowers, R. S. et al., (2015), "Dynamic phototuning of 3D hydrogel stiffness", Proceedings of the National Academy of Sciences, 112(7), 1953-1958), the retina growth rate can be increased locally. The patterning of other mechanical properties such as the viscosity of stress relaxation constants might also be possible. The material may also be patterned using established techniques, such as laser ablation, 3D laser cutting or using photo-tunable viscoelastic hydrogels, for example.

To generate arbitrary organoid shapes, a feedback loop may be implemented with the actual organoid shape as an input and the spatiotemporal mechanical properties of the surrounding material as an output parameter.

(c) Read out the neuronal responses in 3D may be performed in the entire neuronal or retina organoid in three dimensions by using state of the art fluorescence microscopy with calcium sensitive dyes/fluorophores. One example is lightsheet microscopy, an established technique. The technique is ideally suited as it has been optimized for millisecond acquisition speeds (Mickoleit, M. et al.: "High-resolution reconstruction of the beating zebrafish heart", Nature Methods 11, 919-922, 2014) and the imaging of neuronal responses with single neuron resolution (Wolf S. et al.: "Whole-brain functional imaging with two-photon light-sheet microscopy" Nature Methods 12, 379-380, 2015), (Grienberger, C. & Konnerth, A. "Imaging Calcium in Neurons", Neuron 73, 862-885, 2012). To image the neuronal responses of the neuronal organoids, a custom lightsheet microscope is required as commercially available solutions are not able of imaging fast enough in 3D. Using these guiding principles it is possible to achieve the following advantages over the prior art either separately or in combination:
(1) Due to the use of human-like tissue the model system allows for the study of how neuronal responses (e.g. light induced signals) are generated and processed in neuronal tissue such as retina tissue that is close to the human system. Such organoids have a significant better availability and accessibility compared to human tissue explants known from the prior art. Hence, based thereon systematic studies of human neuronal network functions can be performed.
(2) The neuronal organoid allows tuning of neuronal circuits by changes in cell types, densities and the degree of connection, which can be controlled using e.g. the controlled application of transcription factors. In addition, for retina organoids also opto-genetic approaches can be used to tune e.g. the synaptic coupling strength.
(3) In contrast to retina or neuronal organoid explants known from the prior art having limited amount of shapes and organoids growing with uncontrolled shapes, arbitrarily shaped retinas/organoids can be provided.
(4) Due to the use of high-speed fluorescence microscopy, in particular lightsheet microscopy, a three-dimensional readout of neuronal responses of the entire neuronal organoid/retinal tissue can be achieved.

It is a problem of the present disclosure to provide an *in vitro* model system of a neuronal network such as the retina that can be implemented in a measurement device for further studying the network's properties. To this end, there is a need to provide a highly tunable neuronal network sensitive to predetermined influences using a neuronal organoid derived from stem cells, e.g. from human induced pluripotent stem cells. Further, there is a need to control the shape of the organoid, e.g. by spatial and/or temporal control of external influences acting on the organoid. In addition, read-out devices need to be provided that are capable to detect responses of the neuronal network to predetermined influences three-dimensionally and at high acquisition speeds.

It is a problem of the present invention to provide such measurement devices for the use in or the development of medical diagnosis and treatment and/or for the development and/or production of advanced bioinspired electro-optical elements that can be used for signal processing.

The above advantages are achieved and the above problems are solved by the subject-matter of the independent claim.

A measurement device according to the invention comprises neuronal, in particular retinal, tissue grown from stem cells, the neuronal tissue having a three-dimensional shape and neuronal cells that change an electric potential in cells of the neuronal tissue in response to influences that act on the neuronal cells, and a read-out device that is configured to measure neuronal responses of the neuronal tissue via changes in the electric potential generated by the neuronal cells and influencing means configured to exert external influences such as physical, in particular mechanical, and/or optical, and/or chemical influences to the neuronal tissue; wherein the read-out device is configured to measure the neuronal responses of the neuronal tissue, in particular image formation capabilities of the retinal tissue, in response to the external influences exerted by the influencing means; and the influencing means are configured to determine a shape of the neuronal tissue.

Such a measurement device includes the two necessary components to obtain the above advantages. Fully functional neuronal, in particular retinal, tissue grown from stem cells is used according to principally known methods together with a read-out device that allows resolving the changes in electric potential generated by responses of neuronal cells of the neuronal tissue over the whole tissue. This makes it is for the first time possible to obtain detailed insight into neuronal responses of an in principle freely designable, three-dimensional neuronal tissue, i.e. insight into the electrochemical processes that lead to the output of neuronal signals based on which different activities based on neuronal signals are performed by creatures.

The neuronal cells may be photoreceptors of retinal tissue. Then the influences acting on the neuronal cells, i.e. on the photoreceptors may be light that is incident on the photoreceptors. Neuronal responses generated by the photoreceptors may then be image formation capabilities, in particular generation of light-induced signals and their on-site processing. This allows obtaining detailed insight into the processes based on which perception of optical phenomena is performed by creatures.

The neuronal network forming e.g. retinal tissue may be grown from human induced pluripotent stem cells. This allows obtaining insight to the processing of neuronal signals performed in human neuronal networks such as the eyes or the brain that can lead to the development of new types of diagnosis and therapy. Moreover, although the neuronal tissue may in principle be grown from any kind of stem cells the use of induced pluripotent stem cells makes it unnecessary to use embryonal stem cells.

The observed changes in the electric potential are caused by changes in concentration of cytoplasmic calcium ions in cells of the neuronal tissue. These neuronal signals are initiated by the neuronal cells, in particular by retinal photoreceptors. The cells of the neuronal tissue may also comprise a calcium-sensitive fluorescent dye or protein and the read-out device may be configured to measure by high-speed fluorescence microscopy, in particular by light-sheet microscopy, a distribution of the calcium sensitive fluorescent dye or protein within a measured part of the cells of the neuronal tissue, to determine changes in the concentration of cytoplasmic calcium ions within the measured part of the cells from the measured distribution of the calcium sensitive fluorescent dye or protein, and to determine the changes in the electric potential within the measured part of the cells from the determined changes in the concentration of cytoplasmic calcium ions.

By using the techniques of high-speed fluorescence microscopy, e.g. lightsheet microscopy, for the read out of the electric potential, a reliable method for extracting data from the entire neuronal tissue can be provided. In fact, by introducing calcium sensitive fluorescent dyes into the cells of the neuronal tissue, the movements of cytoplasmic calcium ions can be tracked by tracking the fluorescent dyes that will bind to the calcium ions. The readout is done in a known way by light sheet microscopy, where a laser beam focused in only one direction, i.e. to a "light sheet", can be scanned over the whole body of the retinal tissue to excite fluorescence of the fluorescent dyes. This fluorescence can be read out by optical devices, such as microscopes, photomultipliers, photo amplifiers and/or cameras, which are arranged perpendicular to the plane of the light sheet. This method allows reading out the image processing carried out in the neuronal tissue by electrochemical reactions, i.e. by changes in concentration of the calcium ions, with millisecond time resolution over the entire neuronal tissue or at least in cell regions of particular interest such as e.g. the photoreceptor cells, ganglion cells and/or connecting neurons (amacrine cells, horizontal cells and bipolar cells) of retinal tissue. It is therefore for example possible to measure the retina's image formation capabilities, i.e. the signal transport and - processing through the retina from light absorption (photoreceptor cells) to the transmission of the signal to optic nerves (ganglion cells) with high spatial and temporal resolution.

Knowledge about this process in human-like tissues can be used in the development of medical treatments. As an example, the operation of the neuronal network formed by the neuronal tissue can be directly measured as a function of drugs, e.g. activators and inhibitors of neurotransmitters. Moreover, by using retinal tissue the system can be used to design and implement advanced on-site image filtering and processing devices.

The measurement device comprises influencing means that are configured to exert external, i.e. physical, in particular mechanical and/or optical, and/or chemical influences to the neuronal tissue. The read-out device is configured to measure the neuronal responses of the neuronal tissue, in particular image formation capabilities of the retinal tissue, in response to the influences exerted by the influencing means. This allows performing dedicated experiments with the measurement device. For example, the influencing means may be designed as an in principle known optical system that allows projecting of light of predetermined intensity, wavelength and temporal/spatial pattern onto a retinal tissue. Then the dependency of the signals measured by the read-out device and hence the image formation capabilities of the retinal tissues on the incident light can be analyzed in detail. The influencing means may, however, also exert mechanical forces to the retinal tissue or other neuronal tissues such as brain tissue to analyze the changes in the performance of the respective neuronal tissue in response to these mechanical forces. In addition or alternatively, also chemical influences may be measured by analyzing the performance of the neuronal tissue after specific chemical substances such as medicaments have been provided to the neuronal tissue.

By performing such experiments, insight into the development of the eye, in particular of the human eye, or other neuronal organoids such as the brain or the spinal cord under experimentally controllable influences can be obtained *in vitro.* This has the particular advantage that the neuronal tissue can be exposed to specific conditions that can usually not be applied without mixture with other, undesired influences to the respective organoids *in vivo.* This allows separation and classification of effects of different influences, which in turn can be used in the development of diagnostic tools and medical treatments. In addition, gene editing techniques are applicable to *in vitro* systems. This will allow understanding how genetic defects affect organoid functions such as the retina function and helps finding genetic and chemical approaches to tackle these diseases.

The influencing means are configured to determine a shape of the neuronal tissue. This may be done for example by optical supervision of the neuronal tissue. According to another example this may be done by monitoring the mechanical interaction of the neuronal tissue with a surrounding environment or material that forms then a part of the influencing means, such as solid materials, liquids or matrix-like structures.

Hence, in addition to the possibility to observe neuronal responses of the neuronal tissue with the measurement device, such as the image formation capabilities of retinal tissue, it is also conceivable to observe the shape of the neuronal tissue. This has the advantage that also changes in the shape of the neuronal tissue in response to external influences can be observed, if necessary together with corresponding changes in the neuronal network performance. This can be used to further investigate cross-relations between the growth of neuronal tissue such as the retina and external influences, in particular of light on the retina.

In fact, not only couplings in the neuronal network forming the retinal tissue are dependent on the external influences on the retina, such as light, but also the shaping of the retinal tissue can depend on such influences. For example, the presence of neurotransmitter such as dopamine that is generated upon the incidence of light on a retina might have an influence on the development of shortsightedness as it also triggers growth of the retina in specific regions. Hence, observing the relation between experimentally controlled external influences and the shaping of retinal tissue can lead to a deeper understanding of the development process of the eye and of eye diseases that will be essential for the development of new types of medical diagnosis and treatment.

Here, the influencing means may have a known, controllable form, the neuronal tissue may be embedded such in the influencing means that the neuronal tissue and the influencing means interact mechanically with each other, and the shape of the neuronal tissue may be determined from the impact of the mechanical interaction on the influencing means or via optical techniques. To this end, the mechanical interaction of the neuronal tissue with a surrounding environment or material, which forms then a part of the influencing means, such as a highly viscous liquid or a matrix, may be monitored.

For example, changes in the physical states of the influencing means that surrounds the neuronal tissue, such as e.g. the pressure distribution, can be measured by in principal known methods that might however not be applied directly to the neuronal tissue, for example as they might influence the tissue itself or might even destroy it. From the measured mechanical interaction one can then recalculate the shape of the neuronal tissue by applying known algorithms. For example, the pressure distribution in the influencing means and/or its variation can give insight into the shape of the neuronal tissue, as the neuronal tissue causes this pressure distribution at least partly. Hence, filtering the pressure caused by the neuronal tissue from the overall pressure distribution gives insight into the shape of the neuronal tissue.

Major eye diseases such as glaucoma are caused by physical inputs such as mechanical stresses, acting on the retina. Using the measurement device according to the present invention, it is for example possible to measure the retina's function while varying the external stresses. This opens the door to understand better the physical impact on retina diseases and find possible treatments.

The form of the influencing means can be predetermined and may for example be a three-dimensional suspension culture. It may be possible to locally soften or stiffen the material surrounding the neuronal tissue thereby changing the mechanical interaction or influence on the retinal tissue. This can be done by known methods, e.g. by pattering the material with established techniques such as laser ablation or by using the methods and materials suitable for changing mechanical properties of soft materials presented in US 2016/0116394 A1. Hence, changes of the shape of the neuronal tissue with respect to a known initial or intermediate shape can be observed, which improves the measurement results.

The measurement device may further comprise a control unit that is configured to compare the measured neuronal responses and/or the determined shape of the neuronal tissue with predetermined neuronal responses and/or a predetermined shape of the retinal tissue, to generate, based on the comparison, a control signal, and to transmit the control signal to the influencing means. Here, the influencing means are configured to exert physical and/or chemical influences on the neuronal tissue based on the control signal. Using the control unit the measurement device can be used to set up a control loop for bringing the neuronal tissue to a predetermined shape or to predetermined neuronal responses. To this end, the control unit may be capable to use machine learning techniques that allow the control unit to recognize, which external influence leads to a reduction of the difference between the actual form/shape and/or neuronal response/performance of the neuronal tissue and the desired values. Hence, by trying various different external influences the control unit may determine those influences that lead to the desired results. Alternatively or additionally, the control unit may be configured to adjust only a predetermined set of external influences, e.g. light intensity, wavelength and/or pattern, or mechanical force exerted by a surrounding environment having adjustable mechanical properties.

Using such a control loop allows the study of and performance of production of neuronal tissue having predetermined properties such as shape and form of neuronal response. Hence, by using such a control loop for retinal tissue it may be possible to study and/or generate artificial eyes having properties similar or identical to those of humans. In addition, one could design special forms of retinal tissues that are adapted to specific wavelengths and/or intensities of light and could hence serve as constituents of optical elements that convert light into electrical signals. In addition, growing the retina organoid using induced pluripotent stem cells from a patient with a retina disease will allow modelling the disease *in vitro* while measuring the functionality of the patient's retinal neuronal network. Then the control loop can be used to find conditions under which physiological performance can be restored.

The neuronal tissue within the measurement device may have a predetermined initial shape and/or may comprise only a predetermined initial mixture of different cell types, in particular retinal tissue may comprise only a predetermined mixture of retinal photoreceptors. This allows studying neuronal tissue with known initial properties, which will help to better understand influences on the neuronal tissue as the initial conditions can be precisely fixed. For example, it is possible to produce retinal tissue having only a single type of photoreceptor cells by using the right transcription factors during the development of the retina from stem cells. As these conditions do not occur in human retinas, it is e.g. possible to analyze the impact of external influences on single species of photoreceptor cells.

The disclosure further relates to a method for forming organoid, in particular neuronal or retinal, tissue from stem cells with control over the retina shape that comprises developing organoid, in particular neuronal or retina, cells from stem cells, embedding the organoid cells into an environment with controllable mechanical properties, measuring the shape of the organoid cells either using optical techniques or from the impact of a mechanical interaction between the organoid cells and the environment on the environment, comparing the measured shape of the organoid cells with a predetermined shape, inducing tissue growth and/or deformation in predetermined regions by adjusting the mechanical properties of the environment based on the comparison between the measured shape and the predetermined shape such as to minimize a difference between the measured shape and the predetermined shape, and ending the tissue growth and/or deformation after the difference between the measured shape and the predetermined shape is below a predetermined threshold.

In similarity to the above description, organoid, in particular neuronal or retinal, tissue can be generated with a predetermined, controllable form, if the organoid tissue is input in an environment having controllable mechanical properties as for example in materials described in US 2016/0116394 A1. By using such materials as substrate for the organoid cells and a control loop on the shape of the growing organoid cells, it is possible to generate organoid tissue having a predetermined shape. The technique is not limited in its application to the retina organoid, but can also be applied in the case of brain-, lung-, intestine organoids for example.

In developing the organoid cell types only a predetermined mixture of cell types may be developed. As described above, this can for example be achieved by using specific transcription factors during conversion of stem cells into retinal cells. Hence, in addition to the shape also the cellular mixture of the neuronal tissue may be controlled.

In the measurement device a predetermined initial shape of the neuronal tissue and a predetermined initial mixture of different cell types may be obtained by the methods described above.

A neuronal organoid may be formed by using the neuronal organoid cells obtained after ending the growth of organoid cells as neuronal tissue in the measurement device described above in order to further adapt the shape and/or the neuronal responses of the neuronal tissue. This allows to fine tune the shape and/or neuronal responses of the neuronal tissue by performing an adaption of the shape or the interconnection of the neuronal network of the neuronal cells such that desired properties of the neuronal tissue can be obtained.

The disclosure further relates to an artificial eye that may comprise retinal tissue formed according to one of the above described methods. Similarly, an electro-optical element for transforming input light into an electrical signal may comprise retinal tissue formed according to the aforementioned methods. Hence, the above methods and measurement devices may be used to manufacture artificial eyes that can be used in medicine, and electro-optical elements for signal processing, or at least to improve the understanding how to manufacture such devices.

In the following a detailed description of the present invention will be given based on the appended figures. It is illustrated in
**Fig. 1** a schematic view of a measurement device;
**Fig. 2** a schematic view of a measurement device;
**Fig. 3** a schematic view of a measurement device according to an embodiment;
**Fig. 4** a schematic view of influencing means according to an embodiment;
**Fig. 5** a schematic view of a measurement device according to an embodiment;
**Fig. 6** a schematic view of a process flow of a method for forming retinal tissue;
**Fig. 7** a schematic view of a process flow of a method for forming retinal tissue;
**Fig. 8** a schematic view of an artificial eye and
**Fig. 9** a schematic view of an optical element.

In the following description reference is made to retinal tissue that comprises photoreceptors that change an electric potential in cells of the retinal tissue in response to light incident on the photoreceptors. This electric potential is read out to determine image formation capabilities of the retinal tissue.

However, this restriction to retinal tissue is only made for ease of description and is not meant to be limiting. Instead of retinal tissue any neuronal tissue that can be grown from stem cells may be used.

Moreover, any influence acting on such a neuronal tissue will have an effect on neuronal cells within the neuronal tissue that will change the electric potential within the cells in response to this influence. "Change" does here not only comprise a direct and short-term variation of the electric potential caused directly by the influence (such as a signal of a photoreceptor on which light is incident), but also the variation in the electric potential caused by an interconnection of the neuronal tissue's neuronal network that is caused by the influence (such as an interconnection formed under the influence of chemicals and/or drugs or under a mechanical influence).

Accordingly, not only the image formation capabilities of retinal tissue may be determined from the changes in the electric potential, but more generally also neuronal responses constituted by these changes in the electric potential that are caused by the influences. The read-out of image formation capabilities described below is only a specific example that can be easily generalized by a person skilled in the art to the capabilities of other neuronal tissues.

Hence, although described in detail below with respect to retinal tissue the present invention is more generally also applicable to the monitoring and/or tuning of properties of arbitrary neuronal tissues, such properties being for example the shape or the neuronal responses of the neuronal tissue.

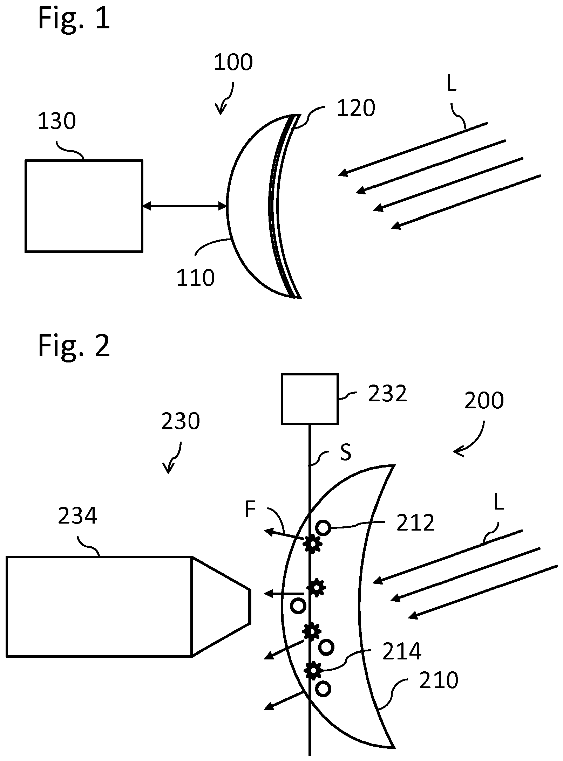

Fig. 1 shows a schematic view of a measurement device 100. The measurement device 100 comprises retinal tissue 110 *in vitro* and a read-out device 130.

The retinal tissue 110 has an arbitrary three-dimensional form and comprises retinal cells that allow the retinal tissue to receive light L and convert the light into electrical signals. The retinal tissue 110 comprises fully functional retinal photoreceptors 120, i.e. photoreceptor cells, on which light L is incident. In response to the incident light L the retinal photoreceptors 120 change an electric potential within cells of the retinal tissue 110, i.e. within the retinal tissue 110. In particular, the retinal photoreceptors 120 respond to incident light L by adjusting a concentration of cytoplasmic calcium ions within them and change hence an electric potential across their cell membranes. This change in electric potential corresponds to an initial electric signal sent out from the photoreceptors 120, i.e. it constitutes a first phase of signal processing carried out by the retinal tissue 110 that allows reconstruction of images out of the received electromagnetic radiation. The initial electric signal generated by the photoreceptors 120 is further transmitted through calcium signaling to other cell layers of the retinal tissue 110, i.e. to connecting neurons (bipolar cells, amacrine cells and horizontal cells) and ganglion cells, where the signal is further processed and filtered until it would be ready to be transferred over optic nerves to a brain. However, as the retinal tissue 110 is provided *in vitro* and not *in vivo* no read out by optic nerves is provided. As detailed below the signal constituted by the changing electric potential within the retinal tissue 110, i.e. within the cells of the retinal tissue 110, will be read out by the read-out device 130.

The retinal tissue 110 is grown from stem cells. Although the measurement device 100 would operate with any kind of retinal tissue 110 of any creature, the retinal tissue is preferably grown from human induced pluripotent stem cells, which allows analysis of human retinal tissue 110 without the need to extract retinal cells from a living human or to use embryonal stem cells. The retinal tissue 110 may be generated from induced pluripotent stem cells according to established techniques as for example described in Zhong, X. et al.: "Generation of three dimensional retinal tissue with functional photoreceptors from human iPSCs" (Nature Communications, 5, 4047 EP-, 2014), Völkner, M. et al.: "Retinal Organoids from Pluripotent Stem Cells Efficiently Recapitulate Retinogenesis" (Stem Cell Reports, 6(4), 525-538, 2016), Eiraku, M. et al.: "Self-organizing optic-cup morphogenesis in three-dimensional culture" (Nature 472, 51 - 56, 2011), WO 2015/109148 A1, CN 103 409 363 A, and US 2016/0333312 A1.

Here, in brief, stem cells are maintained in a two-dimensional culture and transferred to a three-dimensional suspension culture to form aggregates. Then differentiation into neuroepithelium is induced by activating differentiation pathways using a combination of nodal and laminin proteins. The addition of retina differentiation factors causes a differentiation into retina progenitors and their subsequent self-organization to form an optical cup-like structure. Maturation of the retina cells leads then to the development of the fully functional retinal photoreceptors 120. This is one example, other protocols are also possible.

In this process, by using specific transcription factors, the mixture of cell types, in particular of photoreceptor cell types may be adjusted according to the desired measurements to be carried out by the measurement device 100. For example the density of rod and cone photoreceptor cells determines the spectral response of the device (color vision vs. grey scale). In humans the center of the retina has a higher density of cones to enable color vision whereas the retina periphery mainly consists of rod photoreceptor cell. This combination allows central color vision and high signal to noise in grey scales at the periphery. The ratio of rod-to-cone photoreceptor cells and thus the spectral response of the invented measurement device can be tuned by applying inhibitors for molecular signals at specific stages of organoid development.

One example is the application of a Notch inhibitor at specific times of organoid development.

The size and density of amacrine and ganglion cells determines the length scale for spatial filtering processes implemented within the neuronal network. Larger amacrine cells for example are able to process the output signals from further separated photoreceptors allowing to process larger spatial features. One example is the suppression of neuronal signal coming from of a large, slow moving image projected onto the retina. Therefore, tuning the size and density of those cells allows tuning of the implemented filters.

Besides the established methods for growing retinal cells from stem cells, also methods as described below may be used to obtain retinal tissue 110 that may not only have a predetermined mixture of retinal cells, but also a predetermined shape that might differ from the shape obtained by pure self-organization. Hence, the measurement device 100 allows also analyzing of properties of retinal tissue 110 in dependence of the tissues shape.

The electrical signals developing in and/or running through the retinal tissue 110 after incidence of light L on the photoreceptors L, i.e. the changes in electric potential within the retinal tissue that constitute these electrical signals, are measured by the read-out device 130. As the changes in electric potential determine in the end the image that would be formed in a brain, the read-out device 130 measures the image formation capabilities of the retinal tissue 110 by measuring the electric potential. In fact, the image formation capabilities of the retinal tissue 110 will depend on many factors. Besides the composition of types of photoreceptors 120 and the form or shape of the retinal tissue 110, image formation capabilities are also strongly dependent on the interconnection of the neuronal network formed by the cells of the retinal tissue.

For example, the human eye is able to adapt to a broad range of illumination conditions with its retina even being capable of detecting single photons. To adapt to such a decrease in light levels a "pixel size" within the retina is increased by combining the signals of adjacent photoreceptor cells at the cost of reduced spatial resolution. Hence, binning is performed by the retina. To realize this, adjacent photoreceptor cells become coupled through their gap junctions with a coupling strength determined by the concentration of neurotransmitters, such as e.g. dopamine. Hence, the interconnection of the neuronal network of the human eye allows adapting the image formation capabilities to different levels of light illumination.

By measuring the changes in electric potential with the read-out device it is therefore possible to obtain information about image formation capabilities that can in turn be used to better understand the neuronal network of the retinal tissue 110 and its dependence of certain classes of retinal cells, the shape of the tissue and influences acting from outside on the retinal tissue 110.

Therefore, the measurement device 110 provides new insights into the functions of retinal tissue 110, in particular into human retinal tissue 110 obtained from human induced pluripotent stem cells that could not be obtained otherwise and that will enable to develop and/or to apply new diagnostic and therapeutic applications. In particular, the device can be used to measure response function of light illumination and neurotransmitter concentration which also play a role in many eye diseases such as shortsightedness.

The read-out device 130 may, in principle, obtain information about the electric potential within the retinal tissue 110 in arbitrary manners, e.g. by measuring the electric potential directly by highly sensitive electrodes. However, as explained in detail with respect to Fig. 2 a read-out device 230 may also be capable to measure the electric potential by using high-speed fluorescence microscopy, e.g. lightsheet microscopy or laser-scanning 2-photon microscopy.

Fig. 2 shows a measurement device 200 comprising a retinal tissue 210 as described above and the read-out device 230 configured to perform lightsheet microscopy. To this end, the read-out device 230 comprises a light source 232 and a light receiver 234. The light source 232 is configured to emit light S that is focused in only one direction, e.g. by a cylindrical lens or the like, the light S having a predetermined, preferably adjustable, intensity and wavelength, respectively. The light source 232 may for example be a laser light source that focuses the emerging laser light only into one direction to form the "lightsheet" or light plane S. In Fig. 2 the light plane S is illustrated in a top view, i.e. the light plane extends out of the plane of projection of Fig. 2. Although the extension of the light S to the left and to the right is illustrated as negligible in Fig. 2, the lightsheet when viewed from above may also have a "bow-tie shape" with the neck of the bow tie located at a region of interest for the lightsheet microscopy.

What is essential for lightsheet microscopy is that an object to be analyzed is illuminated in a region of interest by the light source 232 across a (virtual) two-dimensional section through the object. The measurement device 200 is therefore configured such that the light S emitted from the light source 232 of the read-out device 230 intersects with a part of the cells of the retinal tissue 210 in a two-dimensional plane through the retinal tissue 210. This part of the cells constitutes an observed or measured part of the cells.

In the measurement device 200 one or several calcium sensitive fluorescents dyes 214 are introduced in the retinal tissue 210. Alternatively, a genetically encoded calciu-sensitive indicator can be used (GCaMP). The dye or the fluorescent protein 214 allows following the movement of calcium ions 212 of the cells of the retinal tissue 210, e.g. by binding to the calcium ions. Examples for calcium sensitive dyes are Fluo-4 (ThermoFisher) or Rhod-2 (ThermoFisher), examples of genetically encoded calcium indicators are GCaMP5.

The wavelength of the light S emitted from the light source 232 is set such that it excites fluorescence of the calcium sensitive fluorescent dye 214. Hence, along the intersection of the light S with the retinal tissue 210 fluorescent light F will be emitted by the dye 214. From the intensity distribution of the fluorescent light F within the two-dimensional intersection plane of the retinal tissue 210 with the light S the distribution of calcium ions can be determined. A change in the distribution of calcium ions that leads to a change in the electric potential is therefore detectable via the observation of the intensity distribution of the fluorescent light F.

To this end, the read-out device comprises the light receiver 234. The light receiver 234 is arranged such that its detection direction is perpendicular to the plane formed by the intersection between the retinal tissue 210 and the light S, in order to assure that the light receiver 234 is configured to observe this plane. For example the light receiver 234 might include an optical microscope, the optical path of which is substantially perpendicular to the lightsheet S, photomultipliers, photo amplifiers and/or camera systems like CCD-cameras, and the necessary optical components to detect the emitted fluorescent light. The focus of the light receiver 234 may be set on the plane of the lightsheet S. The focus may also be set to a layer close to the lightsheet S in an adjustable manner and may be e.g. separated by 5 µm, 10 µm, 50 µm, 100 µm, 500 µm or more from the lighsheet in a direction perpendicular to the lightsheet. This allows analysing also sections through the retinal tissue 210 around the lightsheet without movement of components of the system. Alternatively, a custom-built high-speed laser scanning microscope with a pulsed femtosecond laser can be used.

The received information about the distribution of the calcium sensitive fluorescent dye 214 is then forwarded from the light receiver to a (not shown) processing unit of the measurement device 200. There, changes in the electric potential of the cells of the retinal tissue 210 located along the light sheet S are determined from this information. Note that the processing unit is not necessarily located close to the other components of the measurement device 200. The processing unit may also be a computer or server to which the data obtained from the light receiver 234 are sent.

By moving either the retinal tissue 210 or the light source 232 and the light receiver 234 the complete retinal tissue 210 can be scanned in two-dimensional slices. Here, relative movement may either be linear or may also include rotations. From the obtained two-dimensional slices a complete image of the retinal tissue may be obtained. However, it is also possible to focus on specific areas of the retinal tissue, e.g. to a layer of photoreceptors located at the light incident side (light L) of the retinal tissue 210, to a layer of ganglion cells located at the opposite side of the retinal tissue 210, or to connecting neuronal cells in between. For imaging purposes, the retina organoid will be mounted in a Petri dish embedded either in culture medium and/or in a polymer gel, such as agarose or methylcellulose which is permeable for nutrients. Mounting in other soft materials while providing sufficient amounts of nutrients is also possible.

By using lightsheet microscopy it is therefore possible to visualize the retinal image processing due to propagation of electrical signals caused by changes of cytoplasmic calcium ion concentration in the whole retinal tissue 210. As the temporal and spatial resolution of lightsheet microscopy is e.g. about 100 frames/s (80 to 120 frames/s) and 1 micron (0.8 to 1.2 microns) it is possible to study the propagation of signals through the neuronal network of the retinal tissue 210 and therefore the image formation capabilities of the retinal tissue 210. This will in turn lead to new insights useful in medicine or in the field of neuronal signal processing.

Here, it is clear that the cells of the retinal tissue must be sufficiently transparent to the excitation light S as well as to the fluorescent light F in order to allow excitation and read out of the fluorescent light F throughout the whole retinal tissue 210. This condition is, however, satisfied for the used retinal tissues, excitation and fluorescent wavelengths.

Fig. 2 only represents a schematic diagram of lighsheet microscopy. It is, however, not intended to limit the present invention to the methods and systems of lighsheet microscopy described with respect to Fig. 2. In fact, any other microscopy technique fulfilling the speed and resolution requirements can be used. In particular, any microscopy method that allows detecting the variation in electric potential in and across the retinal cells could be used. For example, one of the methods described in in Mickoleit, M. et al.: "High-resolution reconstruction of the beating zebrafish heart" (Nature Methods 11, 919-922, 2014), Wolf S. et al.: "Whole-brain functional imaging with two-photon light-sheet microscopy" (Nature Methods 12, 379-380, 2015), or Grienberger, C. & Konnerth, A. "Imaging Calcium in Neurons" (Neuron 73, 862-885, 2012) and/or WO 2011/036094 A1.

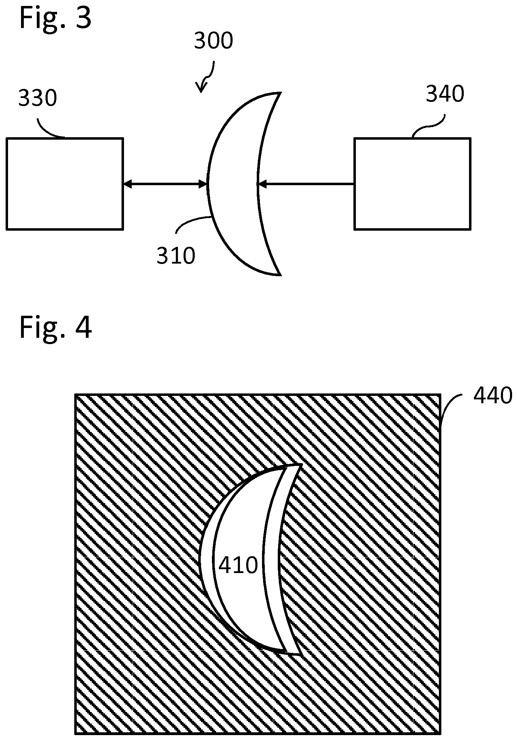

Fig. 3 illustrates schematically a measurement device 300 according to the invention that comprises next to a retinal tissue 310 and a read-out device 330 that correspond to the ones described above, respectively, influencing means 340. The influencing means 340 are coupled such with the retinal tissue 310 that it is possible to exert external influences, i.e. influences that are not generated in the retinal tissue 310, to the retinal tissue 310 in a controllable manner. In turn, the read-out device 330 is capable to measure the changes in the electric potential in the retinal tissue 310 as described above with respect to Figs. 1 and 2 and to determine therefrom changes in the image formation capabilities in dependence from the external influences.

The measurement device 300 allows therefore setting up dedicated experiments for testing the retinal tissue 310 and its image formation capabilities under specific external influences. For example, spatial binning of neuron signals in correlation with dopamine concentration can be analysed by the measurement device 300. In fact, the local dopamine activation, which is e.g. affected by spatiotemporal light patterns on the retinal tissue 310, is assumed to play a major role in the processing of neuronal signals and eye development. Such experiments lead to a deeper understanding of the structure and operation of the retinal tissue and might be used in the development and/or use of new diagnostic and/or therapeutic means. For example, it is possible to characterize by the measurement device 300 quantitatively how illumination patterns control molecular factors in retina tissues, e.g. of humans, at a single cell level. Knowing the dose-response curves (e.g. light-neurotransmitter concentration) within human-like tissues allows the specific design of drugs.

The external influences may be any physical or chemical influences. In particular, the influencing means 340 may consist of an optical apparatus that allows illuminating the entire retinal tissue or parts thereof with light having specific patterns, wavelengths and/or intensities in an in principle known manner. This allows analysing the response of the retinal tissue 310 to specific, experimentally controllable light conditions. Thus, for example the formation of neuronal connections within the retinal tissue 310 in response to specific light conditions can be analysed. This can provide insight into the development of various eye diseases, for which an interrelation between illuminating light patterns and reduced image formation capabilities has been assumed. The optical apparatus may include e.g. a synthetic lens system, in particular an accordingly adapted wide angle lens system, which allows projecting arbitrary light patterns, in particular arbitrary spatiotemporal light patterns, on the retinal tissue 310.

The influencing means 340 may also exert other electromagnetic influences on the retinal tissue 310 than optical influences. For example, also the influence of electric and/or magnetic fields or of electromagnetic radiation outside of the spectrum of visible light may be analysed.

Alternatively or additionally the influencing means 340 may be capable to provide chemical influences to the retinal tissue. For example, the influencing means 340 may allow the provision of chemical components such as medicaments and/or medical substances to the retinal tissue 310 in order to study responses of the neuronal network of the retinal tissue 310 and its image formation capabilities on these chemical components. This allows medical testing of retinal tissue *in vitro* that comes without harm of living creatures. Moreover, it allows a better understanding of the interaction of retinal tissue with various chemical components that may also be present *in vivo* and, hence, to a better understanding of the development and functioning of the eye.

The influencing means 340 may in addition or alternatively also be configured to exert mechanical forces to the retinal tissue 310. For example, the influencing means 340 might be a three-dimensional structure that allows exerting of different pressure values to the retinal tissue 310. Simple means to exert such pressure are for example fixed, non-flexible mechanical components that are pressed onto the retinal tissue 310 such as pins or needles. The retinal tissue 310 may also be brought into an environment as shown in Fig. 4 that presses the retinal tissue 310 in certain, predetermined regions, while no pressure is exerted on other regions. This allows measuring the image formation capabilities and the formation of neuronal networks within the retinal tissue 310 under the influence of specific, predefined mechanical interaction, which also contributes to a deeper understanding of eye development and mechanically induced eye diseases such as glaucoma.

According to the invention, the influencing means 340 are configured such that the shape of the retinal tissue 310 might be measured by the influencing means 340. For example, an optical or electromagnetic influence may be used to determine the shape of the retinal tissue 310 by optic or electromagnetic means. For example, the influencing means 340 may be capable to measure the size and shape of the retinal tissue by detecting light reflected or transmitted by the retinal tissue 310. A three-dimensional form may e.g. be determined by using well known techniques of structured light scanners that may also be directly implemented by the patterns used to test the response of the image formation capabilities of the retinal tissue 310. Alternatively, according to an example not covered by the claimed invention, such an optical detection of the shape of the retinal tissue 310 may also be carried out by the read-out device 330.

In addition or alternatively the influencing means 340 may detect the shape of the retinal tissue 310 by a mechanical back reaction of the retinal tissue 310 on the influencing means 340. In fact, the pressure exerted from the influencing means 340 on the retinal tissue 310 is also exerted from the retinal tissue 310 on the influencing means 340. Hence, a change in the form of the retinal tissue 310 will also alter the mechanical interaction between the retinal tissue 310 and the influencing means 340. The effects of this change in mechanical interaction on the influencing means 340 can be measured in order to deduce changes in the form of the retinal tissue 310. For example, if pressure is exerted by needles or pins on the retinal tissue, changes in the position or form of the needles and pins might be easier to detect than actual changes in the form of the retinal tissue 310.

The retinal tissue 310 may also be embedded in an environment whose mechanical properties, such as e.g. stress distributions within the environment, can be monitored. The shape of a material as a function of space and time, represented by its strain field, is determined by the spatiotemporal stress fields acting on the material and, in addition, the response of the material to these stresses. If both quantities, the stresses and the mechanical properties, are known, the deformation of the material can be calculated. Thus, embedding the organoid in a material with known elastic modulus and known stress distribution allows the determination of its shape in the limit where the mechanics (forces, stresses) of the system are dominated by the external environment. Alternatively, the shape can be directly imaged in 3D using optical techniques such as fluorescence microscopy or brightfield microscopy.

Therefore, by using mechanical interactions, on the one hand the shape of the retinal tissue may be influenced, while on the other hand the resulting changes can be measured immediately. As illustrated schematically in Fig. 4 retinal tissue 410 may be embedded in influencing means 440 that constitute an environment, such as a solid body, a liquid, a matrix or a phase change material, whose mechanical properties, such as elastic modulus and/or viscositiy and/or stress relaxation constants, are controllable. For example, the environment may be a soft material provided with ferrofluid droplets as described in US 2016/0116394 A1. The form of the environment may, however, also be fixed in principle, but irreversibly changed or patterned over time. For example, the local mechanical properties of a solid body or a matrix of fixed initial form, into which the retinal tissue 410 is embedded, may be changed by laser ablation or -cutting using pulsed UV and/or pulsed femtosecond IR lasers. Moreover, local softening or stiffening of environment constituting the influencing means 440 may be performed as e.g. in Stowers, R. S. et al., (2015), "Dynamic phototuning of 3D hydrogel stiffness" (Proceedings of the National Academy of Sciences, 112(7), 1953-1958).

Other established techniques to locally tune the mechanical properties can be used. Examples are polymer gels whose crosslinking can be tuned using optical or chemical techniques. For example, the stiffness of commercially available PEG hydrogels can be controlled using UV light which crosslinks the gel. In this way, the elastic modulus of the gel can be locally increased from about 100 to 10k Pa. Another way to control the elastic modulus of a polymer gel is to control the network strength of its mesh via the local heating of the gel in combination with heat-sensitive crosslinking. In this way, the elastic modulus of the gel can be modulated from about 1kPa to 10kPa. Another way is the use of photodegradable crosslinks which become cleaved upon UV illumination. The elastic modulus can be reduced in a controlled way down to 10% of its initial value with a precision of about 5%.

A change of the mechanical properties of the environment constituting influencing means 440 may result in a situation in which different parts of the retinal tissue 410 are influenced by a different mechanical environment. This is illustrated in Fig. 4 in a schematic manner by showing the upper and lower parts of the retinal tissue 410 in contact with a stiff solid influencing means 440, while the left and right parts of are in contact with the liquid component of the influencing means. However, it should be noted that this is only a simplified exemplary view and that different mechanical interactions may also be present in parts of the retinal tissue 410 that have the same distance to respective neighbouring parts of the influencing means 440.

If the retinal tissue 410 changes its form, this inevitably changes the mechanical interaction with the surrounding environment or influencing means 440. As described above the change in mechanical interaction effects the influencing means 440 such that physical properties of the influencing means 440 such as its form, its internal stress distribution or other mechanical parameters of the influencing means 440 change. This change is then detected and the shape of the retinal tissue 410 is determined therefrom. The processing necessary for this step may be performed by any processor capable thereof internal or external of the influencing means 440.

For example, in Fig. 4 strong mechanic interaction is observed in the upper and lower region of the retinal tissue 410. Hence, pressure in the influencing means 440 next to this region will be high and might lead to a deformation or a change of physical properties, like an index of refraction of the influencing means, which can be measured. To measure the local stresses within the material oil droplets can be used as presented in US 2016/0116394 A1. The local stresses of the material can also be measured for example using 3D traction force microscopy by embedding fluorescent beads.

On the other hand, it may also be possible to control the mechanical properties of the influencing means 440 such that it changes its form or at least the mechanical interaction with the retinal tissue 410. Hence, embedding the retinal tissue 410 in a suitable environment with controllable mechanical properties allows both, determining the shape of the retinal tissue 410 and exerting mechanical influences on the retinal tissue 410. In fact, in Fig. 4 the form of the influencing means 440 allows easy growth and/or movement of retinal tissue to the left and to the right, where the elastic stresses acting on the retina are relatively small (retina-liquid interface), and suppresses growth upwards and downwards, as there the elastic stresses acts against the tissue movement in these regions (retina-solid interface). In this manner, external mechanical forces exerted by the influencing means 440 can bring the retinal tissue 410 practically into any desired three-dimensional shape.

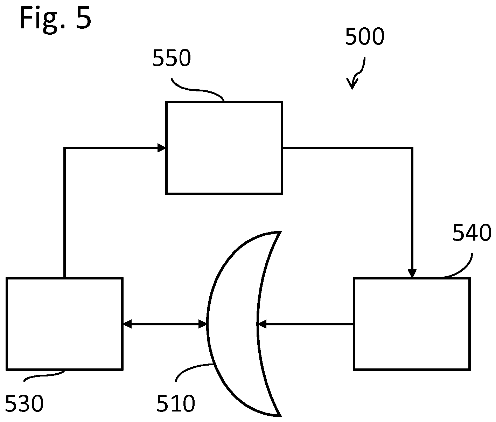

As illustrated in Fig. 5 a measurement device 500 may in addition to a retinal tissue 510, a read-out device 530, and influencing means 540, which correspond to the above described devices, also comprise a control unit 550. The control unit 550 is configured to communicate with the read-out device 530 and the influencing means 540. The control unit 550 may be implemented by a computer or server or any other processor suitable to carry out the below functions. It may be arranged close to the other components of the measurement device 500 or remotely therefrom. Moreover, the control unit 550 may also be combined with any other processing elements of other components of the measurement device 500. For example, all processing functions of the measurement device 500 may be carried out by a single processor or the like.

All measurement results obtained by the measurement device 500, i.e. at least results on the electric potential of the retinal tissue 510 and/or on the shape of the retinal tissue 510, are received by the control unit 550. The control unit 550 compares the obtained measurement results with predetermined values and generates a control signal that is fed to the influencing means 540 to cause the influencing means 540 to exert influences on the retinal tissue 510 that are suitable to bring the afterwards obtained measurement results closer to the predetermined values set or stored in the control unit 550. Hence, the control unit 550 closes a control loop used to adapt the measured properties of the retinal tissue to predetermined set values for these properties.

For example, the control unit 550 may be supplied with predetermined image formation capabilities, as e.g. a certain light sensitivity in a certain spectral region and/or a certain function and performance of the neuronal network. Then read-out image formation capabilities are fed from the read-out device 530 to the control unit 550 and compared by the control unit 550 with the predetermined image formation capabilities. A result of the comparison might e.g. be that light sensitivity in a predetermined spectral region is too low by a certain percentage, e.g. 20%. The control unit 550 may then supply a control signal to the influencing means 540 to trigger an influence that brings measured image formation capabilities and predetermined image formation capabilities closer to each other, i.e. that minimizes a difference between the measured and the predetermined image formation capabilities. In the above example the control signal may trigger influences that lead to an increase in light sensitivity in the desired wavelength region, e.g. by only supplying light having the desired wavelength or by any other suitable measure. Generation of influences is ended, after the difference between the measured and the predetermined image formation capabilities lies below a predetermined threshold. In the above example this might be a difference between desired and obtained light sensitivity of e.g. 1 %, 5 %, 10 % or the like.

Similarly, the shape of the retinal tissue as measured within the measurement device 500 by the influencing means 540 or the read-out device 530 may be used as input parameter for the control unit 550. The control unit 550 produces then a control signal that triggers the influencing means 540 to change influences exerted on the retinal tissue 510 such that the shape of the retinal tissue 510 comes closer to a predetermined shape of the retinal tissue 510 stored in or supplied to the control device 550.

For example, if in Fig. 4 growth of the retinal tissue in the upper region is desired, the control unit 550 may advise the influencing means 440 to change its mechanical properties such that the mechanical interaction between retinal tissue 410 and influencing means 440 becomes smaller in this upper region, e.g. by making the influencing means softer in this region or by changing its form such that a distance between retinal tissue 410 and influencing means 440 becomes larger. Then growth into this region is also allowed. In contrast, by making the gap in the influencing means 440 narrower in the left-right-direction, growth of the retinal tissue 410 in this region will be additionally suppressed.

Hence, the control unit may also be used to bring the form of the retinal tissue 510 close to a desired, predetermined form. For example, if the shape of the retinal tissue is sufficiently close to the desired shape, e.g. if the difference is only a few percent, such as 1%, 5%, 10%, of the overall size of the retinal tissue, the method for generating the retinal tissue is ended.

The control unit 550 may also be configured to obtain the right parameters for a reduction of the difference between measured and predetermined values, such as image formation capabilities or retinal shape, by using machine learning techniques. To this end, the control unit 550 may test various different influences on the retinal tissue 510 and the corresponding changes in the retinal tissue 510. From these results the control unit 550 may be able to deduce by commonly known machine learning techniques which influences must be exerted in order to minimize the difference. In this way not only retinal tissue 510 having desired properties is obtained, but it is also possible to understand the interrelation between the formation of these properties and the influences necessary to develop them, e.g. the interrelation of retina shape and retina image formation capabilities. This insight can then be used in the development of new medicaments or therapies for eye diseases.

As described with respect to Figs. 1 to 5 measurement devices allow to analyse retinal tissue grown from human induced pluripotent stem cells to a deep level *in vitro.* Further, they allow modifying the properties of the retinal tissue by providing specifically controlled influences on the retinal tissue that might lead to the formation of specific, desired properties.

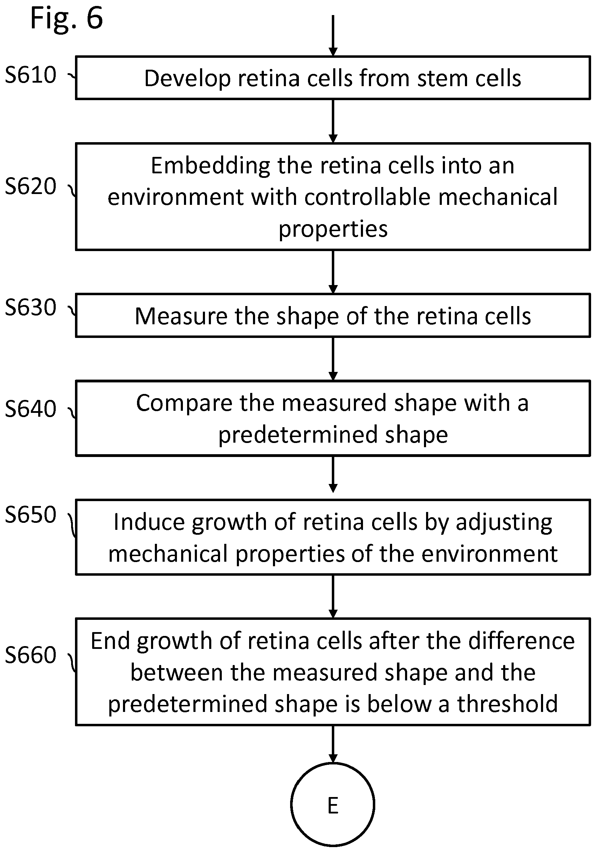

Here, the techniques for influencing the properties of retinal tissue may not only be used for retinal tissue already implemented in measurement devices as described above, but also in the initial growth of the retinal tissue or other organoid or neuronal tissue from stem cells. An exemplary process flow of an according method related to retinal tissue is schematically illustrated in Fig. 6. It is to be understood that this intends no limitation on the use of the method for forming other organoid or neuronal tissue such as brain, lung, or intestine organoids for example. This is achieved by replacing any reference to retina cells below by a reference to respective organoid cells.

At S610 retina cells are developed from stem cells according to well established techniques (Zhong, X. et al.: "Generation of three dimensional retinal tissue with functional photoreceptors from human iPSCs" (Nature Communications, 5, 4047 EP-, 2014), Völkner, M. et al.: "Retinal Organoids from Pluripotent Stem Cells Efficiently Recapitulate Retinogenesis" (Stem Cell Reports, 6(4), 525-538, 2016), Eiraku, M. et al.: "Self-organizing optic-cup morphogenesis in three-dimensional culture" (Nature 472, 51 - 56, 2011), WO 2015/109148 A1, CN 103 409 363 A, and US 2016/0333312 A1).

At S620 the obtained retina cells are embedded into an environment with controllable mechanical properties and at S630 the shape of the retina cells is measured either from the impact of a mechanical interaction between the retina cells and the environment on the environment or by other imaging techniques, e.g. optical imaging techniques. The properties of the environment and the manner in which the measurement of the shape is performed correspond here to the interaction between retinal tissue and influencing means that was described above with respect to Figs. 4 and 5.

At S640 the measured shape of the retina cells is compared with a predetermined shape, e.g. by a control unit as described with respect to Fig. 5.

At S650 growth of retina cells and/or deformation of the retinal tissue in predetermined regions are induced by adjusting the mechanical properties of the environment based on the comparison between the measured shape and the predetermined shape such as to minimize a difference between the measured shape and the predetermined shape. This is done by changing mechanical properties of the environment surrounding the retinal cells as was described above with respect to Figs. 4 and 5.

At S660 the growth of retina cells and/or the deformation of the retinal tissue are ended, when the difference between the measured shape and the predetermined shape is below a predetermined threshold. Hence, if the shape of the retinal tissue is sufficiently close to the desired shape, e.g. if the difference is only a few percent, such as 1%, 5%, 10%, of the overall size of the retinal tissue, the method for generating the retinal tissue is ended.

In this manner retinal tissues, e.g. for the use in measurement devices as described above, can be produced that have already a predetermined form. Moreover, by selecting during development of the retina cells from stem cells the type or mixture of retinal cells, in particular of retinal photoreceptors, to a desired type or mixture, it is also possible to develop retinal tissue that has not only a predetermined shape, but also predetermined image formation capabilities. Such retinal tissue provides an ideal starting ground for the conduction of experiments, as the initial properties of the retinal tissue can be clearly defined, which allows easy detection of responses of the retinal tissue to external influences.

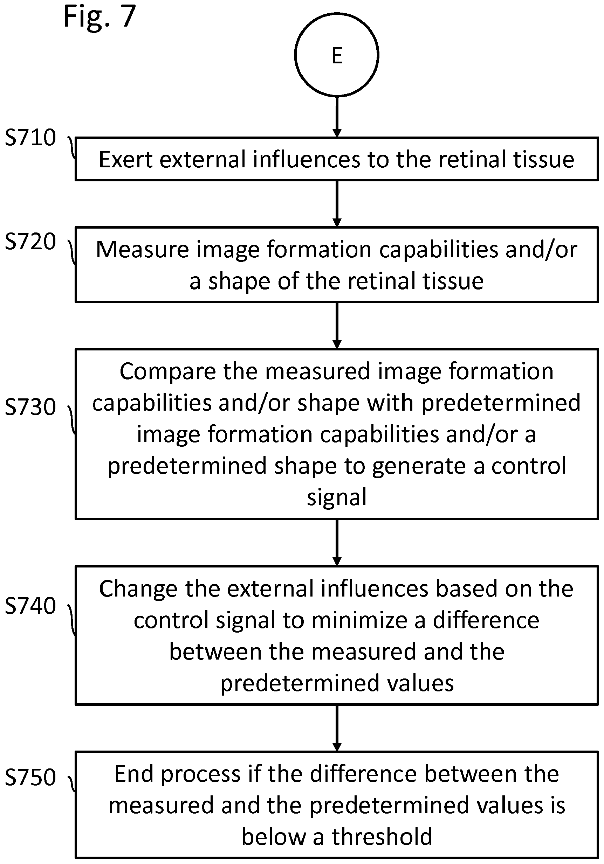

After setting up the initial retinal tissue according to the method described with respect to Fig. 6, this retinal tissue can be used in measurement devices as described with respect to Figs. 1 to 5. According to a method for producing retinal tissue, a process flow of which is schematically illustrated in Fig. 7, the properties of the retinal tissue can be further adjusted by implementing it in measurement devices as described above, where a control loop is used to further adjust the properties.

Here, at S710 external influences are exerted to the retinal tissue and properties of the retinal tissue such as image formation capabilities and/or a shape of the retinal tissue are measured at S720 as described above with respect to Figs. 3 to 5. At S730 these measured properties are compared to predetermined properties, i.e. to a predetermined shape and/or predetermined image formation capabilities. Based on the comparison a control signal is generated.

At S740 the external influences are changed based on the control signal such as to minimize a difference between the measured and the predetermined values as described above with respect to Fig. 5. At S750 the process is ended, if the difference between the measured and the desired properties is acceptable, i.e. if the difference is below a predetermined threshold.

Hence, by the above method it is possible to develop retinal tissue that has desired properties. Moreover, the process of obtaining the retinal tissue may provide further insight, for example, if it is carried out by a control unit capable of using machine learning techniques.

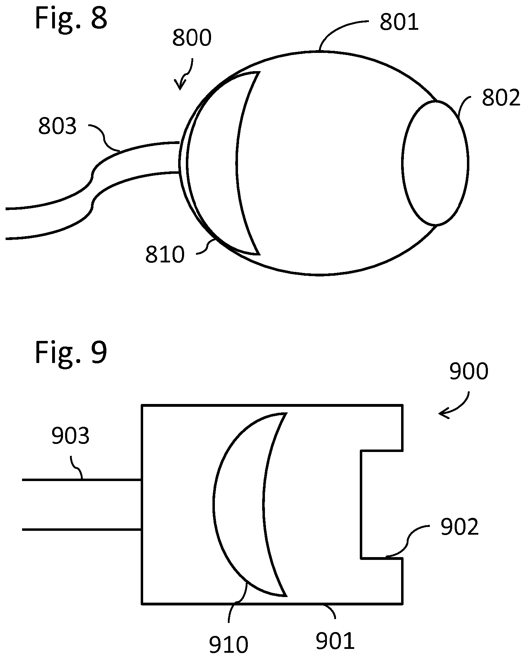

Fig. 8 illustrates schematically an artificial eye 800 that comprises retinal tissue 810 that has been obtained or that was developed according to one of the aforementioned methods. Here, "retinal tissue" comprises also any electronic neuronal network that is designed according to the insights into the neuronal network of retinal tissue scrutinized by measurement devices as described above. The retinal tissue 810 is arranged such in an eye body 801 comprising an eye lens 802 that it receives light through the eye lens 802, converts the light into neuronal signals that are then led via an optic nerve 803 to the brain. Just like the retinal tissue 810 also all other components of the artificial eye do not need to be purely biological tissue, but may be any combination of electronic components, inorganic components, plastic material and/or organic components.

Hence, retinal tissue 810 that is grown from stem cells or that is designed according to insights into neuronal networks obtained by studying such retinal tissue can be used in artificial eyes that might eventually allow blind people to see.

Similarly, as illustrated schematically in Fig. 9 retinal tissue 910 may be part of an electro-optical element 900 that comprises an element body 901, an entry window 902, and a data line 903. The electro-optical element 900 converts light signals incoming through the entry window 902 into electrical signals by the retinal tissue 910, which are then fed and read out via the data line 903. Also here the term "retinal tissue" refers to either biological retinal tissue grown from stem cells by one of the above methods or to an electronic neuronal network designed according to such tissue.

Hence, retinal tissue 910 grown from stem cells or the insights therefrom may also be used in elements for signal processing and may allow the construction of photodetectors that operate according to the principles of the human eye.

## Claims

1. A measurement device (300) comprising:
neuronal, in particular retinal, tissue (310) grown from stem cells, the neuronal tissue (310) having a three-dimensional shape and neuronal cells that change an electric potential in cells of the neuronal tissue (310) in response to influences that act on the neuronal cells;
a read-out device (330) that is configured to measure neuronal responses of the neuronal tissue (310) via changes in the electric potential generated by the neuronal cells; and
influencing means (340) configured to exert external influences such as physical, in particular mechanical, and/or optical, and/or chemical influences to the neuronal tissue (310); wherein
the read-out device (330) is configured to measure the neuronal responses of the neuronal tissue (310), in particular image formation capabilities of the retinal tissue (310), in response to the external influences exerted by the influencing means (340); and
the influencing means (340) are configured to determine a shape of the neuronal tissue (310).

2. The measurement device (300) according to claim 1, wherein
the neuronal cells are photoreceptors (120);
the influences acting on the neuronal cells is light (L) incident on the photoreceptors (120); and
the neuronal responses are image formation capabilities, in particular generation of light-induced signals and their on-site processing.

3. The measurement device (300) according to one of the preceding claims, wherein the neuronal tissue (310) is grown from human induced pluripotent stem cells.

4. The measurement device (300) according to one of the preceding claims; wherein
the changes in the electric potential are caused by changes in concentration of cytoplasmic calcium ions (212) in cells of the neuronal tissue (210) initiated by the neuronal cells, in particular by retinal photoreceptors (310);
the cells of the neuronal tissue comprise a calcium-sensitive fluorescent dye or protein (214); and
the read-out device (330) is configured
to measure by high-speed fluorescence microscopy, in particular by light-sheet microscopy, a distribution of the calcium sensitive fluorescent dye or protein (214) within a measured part of the cells of the neuronal tissue (310),
to determine changes in the concentration of cytoplasmic calcium ions (212) within the measured part of the cells from the measured distribution of the calcium sensitive fluorescent dye or protein (214), and
to determine the changes in the electric potential within the measured part of the cells from the determined changes in the concentration of cytoplasmic calcium ions (212).

5. The measurement device (300) according to any one of the preceding claims, wherein
the influencing means (440) have a known, controllable form;
the neuronal tissue (410) is embedded such in the influencing means (440) that the neuronal tissue (410) and the influencing means (440) interact mechanically with each other; and
the shape of the neuronal tissue (410) is determined from the impact of the mechanical interaction on the influencing means (440) or via optical techniques.

6. The measurement device (500) according to any one of the preceding claims, further comprising:
a control unit (550) that is configured to compare the measured neuronal responses and/or the determined shape of the neuronal tissue (510) with predetermined neuronal responses and/or a predetermined shape of the neuronal tissue (510), to generate, based on the comparison, a control signal, and to transmit the control signal to the influencing means (540); wherein
the influencing means (540) are configured to exert physical and/or chemical influences on the neuronal tissue (510) based on the control signal.

7. The measurement device (300) according to any one of the preceding claims, wherein
the neuronal tissue (310) has a predetermined initial shape and/or comprises only a predetermined initial mixture of different cell types.

## Patentansprüche

1. Messgerät (300), aufweisend:
neuronales, insbesondere die Netzhaut betreffendes, aus Stammzellen gewachsenes Gewebe (310), wobei das neuronale Gewebe (310) eine dreidimensionale Form und neuronale Zellen aufweist, die als Antwort auf Einflüsse, die auf die neuronalen Zellen wirken, ein elektrisches Potential in Zellen des neuronalen Gewebes (310) ändern,
ein Auslesegerät (330), das dafür konfiguriert ist, neuronale Antworten des neuronalen Gewebes (310) über Änderungen im elektrischen Potential, die durch die neuronalen Zellen erzeugt werden, zu messen, und
Beeinflussungsmittel (340), die dafür konfiguriert sind, externe Einflüsse wie etwa physikalische, insbesondere mechanische, und/oder optische und/oder chemische Einflüsse auf das neuronale Gewebe (310) auszuüben, wobei
das Auslesegerät (330) dafür konfiguriert ist, die neuronalen Antworten des neuronalen Gewebes (310), insbesondere Abbildungsfähigkeiten des die Netzhaut betreffenden Gewebes (310), als Antwort auf die durch die Beeinflussungsmittel (340) ausgeübten externen Einflüsse zu messen, und
die Beeinflussungsmittel (340) dafür konfiguriert sind, eine Form des neuronalen Gewebes (310) zu bestimmen.

2. Messgerät (300) nach Anspruch 1, wobei
die neuronalen Zellen Fotorezeptoren (120) sind,
die auf die neuronalen Zellen wirkenden Einflüsse auf die Fotorezeptoren (120) einfallendes Licht (L) sind und
die neuronalen Antworten Abbildungsfähigkeiten, insbesondere eine Erzeugung lichtinduzierter Signale und ihre Vor-Ort-Verarbeitung, sind.

3. Messgerät (300) nach einem der vorhergehenden Ansprüche, wobei
das neuronale Gewebe (310) aus humaninduzierten pluripotenten Stammzellen gewachsen ist.

4. Messgerät (300) nach einem der vorhergehenden Ansprüche, wobei
die Änderungen im elektrischen Potential durch Änderungen in der Konzentration zytoplasmatischer Calciumionen (212) in Zellen des neuronalen Gewebes (210) verursacht werden, die durch die neuronalen Zellen, insbesondere durch die die Netzhaut betreffenden Fotorezeptoren (310), initiiert werden,
die Zellen des neuronalen Gewebes einen calcium-empfindlichen fluoreszierenden Farbstoff oder ein Protein (214) aufweisen und
das Auslesegerät (330) dafür konfiguriert ist,
mittels einer Hochgeschwindigkeits-Fluoreszenzmikroskopie, insbesondere mittels einer Lichtscheibenmikroskopie, eine Verteilung des calcium-empfindlichen fluoreszierenden Farbstoffs oder Proteins (214) innerhalb eines gemessenen Teils der Zellen des neuronalen Gewebes (310) zu messen,
Änderungen in der Konzentration zytoplasmatischer Calciumionen (212) innerhalb des gemessenen Teils der Zellen aus der gemessenen Verteilung des calcium-empfindlichen fluoreszierenden Farbstoffs oder Proteins (214) zu bestimmen und
die Änderungen im elektrischen Potential innerhalb des gemessenen Teils der Zellen aus den bestimmten Änderungen in der Konzentration zytoplasmatischer Calciumionen (212) zu bestimmen.

5. Messgerät (300) nach einem der vorhergehenden Ansprüche, wobei die Beeinflussungsmittel (440) eine bekannte steuerbare Form haben, das neuronale Gewebe (410) so in den Beeinflussungsmitteln (440) eingebettet ist, dass das neuronale Gewebe (410) und die Beeinflussungsmittel (440) mechanisch miteinander wechselwirken, und
die Form des neuronalen Gewebes (410) aus der Einwirkung der mechanischen Wechselwirkung auf die Beeinflussungsmittel (440) oder über optische Techniken bestimmt wird.

6. Messgerät (500) nach einem der vorhergehenden Ansprüche, ferner aufweisend:
eine Steuerungseinheit (550), die dafür konfiguriert ist, die gemessenen neuronalen Antworten und/oder die bestimmte Form des neuronalen Gewebes (510) mit vorbestimmten neuronalen Antworten und/oder einer vorbestimmten Form des neuronalen Gewebes (510) zu vergleichen, um basierend auf dem Vergleich ein Steuerungssignal zu erzeugen und das Steuerungssignal zu den Beeinflussungsmitteln (540) zu übertragen, wobei
die Beeinflussungsmittel (540) dafür konfiguriert sind, basierend auf dem Steuerungssignal physikalische und/oder chemische Einflüsse auf das neuronale Gewebe (510) auszuüben.

7. Messgerät (300) nach einem der vorhergehenden Ansprüche, wobei
das neuronale Gewebe (310) eine vorbestimmte Anfangsform hat und/oder nur eine vorbestimmte Anfangsmischung unterschiedlicher Zelltypen aufweist.

## Revendications

1. Dispositif de mesure (300) comprenant :
un tissu neuronal (310), en particulier rétinien, cultivé à partir de cellules souches, le tissu neuronal (310) ayant une forme à trois dimensions et des cellules neuronales qui changent un potentiel électrique dans des cellules du tissu neuronal (310) en réponse à des influences qui agissent sur les cellules neuronales ;
un dispositif de lecture (330) qui est configuré pour mesurer des réponses neuronales du tissu neuronal (310) via des changements de potentiel électrique générés par les cellules neuronales ; et
des moyens influenceurs (340) configurés pour exercer des influences externes telles que des influences physiques, en particulier mécaniques, et/ou optiques, et/ou chimiques sur le tissu neuronal (310) ; dans lequel
le dispositif de lecture (330) est configuré pour mesurer les réponses neuronales du tissu neuronal (310), en particulier des capacités de formation d'image du tissu rétinien (310), en réponse aux influences externes exercées par les moyens influenceurs (340) ; et
les moyens influenceurs (340) sont configurés pour déterminer une forme du tissu neuronal (310).

2. Dispositif de mesure (300) selon la revendication 1, dans lequel les cellules neuronales sont des photorécepteurs (120) ;
les influences agissant sur les cellules neuronales sont la lumière (L) incidente sur les photorécepteurs (120) ; et
les réponses neuronales sont des capacités de formation d'image, en particulier la production de signaux induits par la lumière et leur traitement sur site.

3. Dispositif de mesure (300) selon l'une des revendications précédentes, dans lequel
le tissu neuronal (310) est cultivé à partir de cellules souches pluripotentes induites humaines.

4. Dispositif de mesure (300) selon l'une des revendications précédentes ; dans lequel
les changements de potentiel électrique sont causés par des changements de concentration d'ions de calcium cytoplasmique (212) dans des cellules du tissu neuronal (210) initiés par les cellules neuronales, en particulier par des photorécepteurs rétiniens (310) ;
les cellules du tissu neuronal comprennent un colorant ou protéine (214) fluorescent sensible au calcium ; et
le dispositif de lecture (330) est configuré
pour mesurer par microscopie de fluorescence haute vitesse, en particulier par microscopie à nappe de lumière, une distribution du colorant ou de la protéine (214) fluorescent sensible au calcium à l'intérieur d'une partie mesurée des cellules du tissu neuronal (310),
pour déterminer des changements de concentration d'ions de calcium cytoplasmique (212) à l'intérieur de la partie mesurée des cellules à partir de la distribution mesurée du colorant ou de la protéine (214) fluorescent sensible au calcium, et
pour déterminer les changements de potentiel électrique à l'intérieur de la partie mesurée des cellules à partir des changements déterminés de la concentration d'ions de calcium cytoplasmique (212).

5. Dispositif de mesure (300) selon l'une quelconque des revendications précédentes, dans lequel
les moyens d'influence (440) ont une forme connue, apte à être commandée ;
le tissu neuronal (410) est intégré dans les moyens d'influence (440) de telle sorte que le tissu neuronal (410) et les moyens d'influence (440) interagissent mécaniquement entre eux ; et
la forme du tissu neuronal (410) est déterminée à partir de l'impact de l'interaction mécanique sur les moyens d'influence (440) ou via des techniques optiques.

6. Dispositif de mesure (500) selon l'une quelconque des revendications précédentes, comprenant en outre :
une unité de commande (550) qui est configurée pour comparer les réponses neuronales mesurées et/ou la forme déterminée du tissu neuronal (510) à des réponses neuronales mesurées prédéterminées et/ou à une forme déterminée du tissu neuronal (510), pour générer, sur la base de la comparaison, un signal de commande, et pour transmettre le signal de commande aux moyens d'influence (540) ; dans lequel
les moyens d'influence (540) sont configurés pour exercer des influences physiques et/ou chimiques sur le tissu neuronal (510) sur la base du signal de commande.

7. Dispositif de mesure (300) selon l'une quelconque des revendications précédentes, dans lequel
le tissu neuronal (310) a une forme initiale prédéterminée et/ou comprend seulement un mélange initial prédéterminé de types de cellules différents.
